Europäisches Patentamt

European Patent Office

Office européen des brevets

(19)

(11) Veröffentlichungsnummer: **0 125 634**
**B1**

(12) **EUROPÄISCHE PATENTSCHRIFT**

(45) Veröffentlichungstag der Patentschrift: 26.09.90

(51) Int. Cl.⁵: **A 61 K 45/08**, A 61 K 9/22

(21) Anmeldenummer: **84105287.1**

(22) Anmeldetag: **10.05.84**

(54) **Verwendung einer sekretolytisch wirkenden Substanz zur Herstellung eines Antischnarchmittels und zur Bekämpfung des Schnarchphänomens.**

(30) Priorität: **13.05.83 DE 3317530**
**13.05.83 DE 3317558**

(43) Veröffentlichungstag der Anmeldung:
**21.11.84 Patentblatt 84/47**

(45) Bekanntmachung des Hinweises auf die Patenterteilung:
**26.09.90 Patentblatt 90/39**

(84) Benannte Vertragsstaaten:
**AT BE CH DE FR GB IT LI NL SE**

(56) Entgegenhaltungen:
**EP-A-0 053 754**
**EP-A-0 069 259**

**ROTE LISTE, 1982, Editio Cantor, Nr. 23 137 - Nr 23 148, Aulendorf/Württ, DE**

**Die Akte enthält technische Angaben, die nach dem Eingang der Anmeldung eingereicht wurden und die nicht in dieser Patentschrift enthalten sind.**

(73) Patentinhaber: **Reichert, Dietrich, Dr. med.
Apartado de Correos 773
E-07800 Ibiza/Ibiza Baleares (ES)**

(72) Erfinder: **Reichert, Dietrich, Dr. med.
Apartado de Correos 773
E-07800 Ibiza/Ibiza Baleares (ES)**

(74) Vertreter: **Hansen, Bernd, Dr.rer.nat. et al
Hoffmann, Eitle & Partner Patentanwälte
Arabellastrasse 4 Postfach 81 04 20
D-8000 München 81 (DE)**

## Beschreibung

Die Erfindung betrifft die Verwendung einer sekretolytisch und/oder sekretoproduktiv wirkenden Substanz zur Herstellung eines Mittels zur therapeutischen Behandlung gesundheitsschädlichen Schnarchens und die Verwendung einer sekretolytisch und/oder sekretoproduktiv wirkenden Substanz gegen störendes Schnarchen.

Das Schnarchen ist ein Phänomen, das auf einer rasselnden Atmung beruht, die beim Menschen während des Schlafes auftreten kann. Wegen der dadurch enstehenden Belästigung, die das Schnarchen für Mitmenschen bedeutet, sind bisher zahlreiche Versuche unternommen worden, diesem Phänomen abzuhelfen.

Als Ursache des Schnarchens wurden Obstruktionen (Unebenheiten) im Bereich der oberen Luftwege festgestellt. Beim Vorgang des Ein- und Ausatmens wird die Luft über Komplexe Strömungswege geführt. Unebenheiten im Bereich dieser Strömungswege führen notwendigerweise zu Turbulenzen in der Luftströmung. Dadurch ergibt sich eine Atmungsbehinderung, die unterhalb der Bewusstseinsschwelle liegt. Die Behinderungen im Strömungsweg und die dadurch verursachten Turbulenzen haben zur Folge, dass örtlich ein Unterdruck (Sog) entsteht. Dieser Unterdruck führt zu flatternden Bewegungen von weichen, erschlafften Strukturen im Bereich des Luftströmungsweges. Insbesondere kommt es zu einer Hin- und Herbewegung des weichen Gaumens infolge der genannten Turbulenzen.

Obwohl zur Verhinderung des Schnarchens bereits zahlreiche Untersuchungen durchgeführt worden sind, haben sie bisher nicht zu dem gewünschten Erfolg geführt. Man ist dabei von Mitteln ausgegangen, die auf der Grundlage von Chemotherapeutika oder Antibiotika, Corticoiden oder Antihistaminika aufgebaut waren. Diese Mittel haben sich jedoch als nicht wirksam genug erwiesen, um das Schnarchen auf Dauer zu verhindern, oder sie führten bei längerer Einnahme zu einer Schädigung der Nasen- und Rachenschleimhaut. Dies trifft auch für die aus früheren Zeiten bekannten Versuche mit etherischen Ölen, wie z.B. Menthol, Kamille, Eukalyptusöl, etc., in höheren Konzentrationen zu. Erst in neuerer Zeit konnte gezeigt werden, das Obstruktionen, die als Ursache des Schnarchens angesehen werden können, insbesondere durch Austrocknen der Schleimhäute oder zusätzliche trockene Schleimfelder mit Mikrorissen, durch Ablagerungen von zähem Schleim etc. entstehen.

Die europäische Patentanmeldung 0053754 des gleichen Anmelders beschreibt ein Mittel zur Bekämpfung des Schnarchens, das einen oberflächenaktiven Stoff, ein Konservierungsmittel und eine die Schleimhäute geschmeidig machende Substanz in physiologischer Kochsalzlösung enthält. Dieses Mittel soll das Austrocknen der Schleimhäute während der Nacht verhindern. Hierzu ist es erforderlich, vor dem Einschlafen eine bestimmte Menge des Mittels in den Nasen-Rachenraum einzuträufeln.

Die aufgabe der vorliegenden Erfindung liegt im Auffinden eines geeigneten Mittels zur Bekämpfung des Schnarchens, womit insbesondere auch hartnäckige Fälle von Schnarchen unterbunden werden können, ohne dass sich dabei schädliche Nebenwirkungen durch Beeinträchtigung der Nasen- und Rachenschleimhaut einstellen. Insbesondere soll dabei das Schnarchgeräusch beim ''Common-Schnarchen'' unterdrückt werden.

Diese Aufgabe wird gemäss der Erfindung gelöst durch die Verwendung einer sekretolytisch und/oder sekretoproduktiv wirkenden Substanz neben üblichen pharmazeutisch verträglichen Trägersubstanzen oder Streck- bzw. Verdünnungsmitteln zur Herstellung eines Mittels zur therapeutischen Behandlung gesundheitsschädigenden Schnarchens in einer oralen Verabreichungsform, die einen intensiven Kontakt des Wirkstoffes mit der Rachenschleimhaut ausschliesst.

Im weiteren umfasst der Erfindung die Verwendung einer sekretolytisch und/oder sekretoproduktiv wirkenden Substanz gegen störendes Schnarchen, in einer oralen Verabreichungsform, die einen intensiven Kontakt des Wirkstoffes mit der Rachenschleimhaut ausschliesst.

Bevorzugt ist gemäss der Erfindung die Verwendung einer wirksamen Dosis eines Sekretolytikums.

Das erfindungsgemäss verwendete Antischnarchmittel zeichnet sich insbesondere durch einen wirksamen Gehalt eines die Funktion der Schleimhautdrüsen des Atemtraktes anregenden Wirkstoffes neben üblichen Trägersubstanzen bzw. Verdünnungsmitteln aus.

Besonders vorteilhaft ist es, wenn das erfindungsgemäss verwendete Sekretolytikum über die folgenden Eingeschaften verfügt:

a) Regulierung und Normalisierung der Schleimviskosität,

b) Senkung der Schleimadhäsion durch Aktivierung körpereigener, oberflächenaktiver Eigenschaften der Sekretion,

c) Stimulierung der serösen Schleimproduktion, und

d) Aktivierung der Tätigkeit der mukoziliaren Funktion.

Erfindungsgemäss geeignete Sekretolytika sind im Stand der Technik bekannt. Diese bekannten Sekretolytika finden Anwendung bei der Behandlung von Erkrankungen des Respirationsstraktes, die mit pathologisch veränderter Sekretbildung einhergehen, wobei sie teilweise durch Inhalieren oder Instillieren, teilweise in Form von Tabletten etc. oder Säften oral verabreicht werden.

Gemäss der Erfindung wurde nun überraschenderweise gefunden, dass sich durch Anwendung geeigneter Sekretolytika das Schnarchen, sei es gesundheitsschädigend oder, wie vorstehend bereits beschrieben, ein für Mitmenschen lästiges Phänomen, verhindert bzw. vermindert werden kann.

Die oral eingenommenen Sekretolytika führen zu einer Anregung der Schleimhautdrüsen im Atemtrakt, wodurch sich eine Verflüssigung von zähem Schleim im Nasen- und Rachenraum und/oder eine Anregung der Schleimsekretion ergibt, wodurch das Austrocknen der Schleimhäute und die Bildung von Mikrorissen verhindert werden.

Zu den bekannten und gemäss der Erfindung geeigneten Sekretolytika zählen u.a. die Verbindungen Bromhexin, Ambroxol, Eprazinon, Carbocistin, N-Acetyl-L-cystein sowie Saponine, die z.B. in Radix Senagae, Radix Primularae, Radix Saponariae und Radix Liquiritiae enthalten sind, ebenso wie kohlenhydrathaltige Inhaltsstoffe aus Radix altae, Lichen Islandicus, Folia Malvae oder Carrageen; besonders bevorzugt sind Bromhexin und Ambroxol.

Die vorstehend genannten Sekretolytika können als solche oder in Form ihrer pharmazeutisch verträglichen Salze eingesetzt werden.

Das orale Antischnarchmittel gemäss der Erfindung kann in verschiedenen galenischen Formen verabreicht werden. Besonders bevorzugt sind zur Erzielung einer Langzeitwirkung Tabletten, Dragees, Kapseln, Komprimate, Granulate, Mikrokapseln etc. Daneben ist es aber auch möglich, das orale Antischnarchmittel in Form von Tropfen oder als Saft zu verabreichen.

Die an einen Schnarchenden zu verabreichende Gesamtdosis des Wirkstoffes richtet sich nach der jeweiligen Effizienz des eingesetzten Wirkstoffes sowie der Anwendungsform und liegt im allgemeinen im Bereich zwischen 5 und 500 mg, bevorzugt zwischen 10 und 100 mg.

Im Falle der Verabreichung von Ambroxol liegt der besonders bevorzugt Bereich bei einer Wirkstoffdosis von 30 bis 100, bevorzugt von 50 bis 100 mg. Versuche unter Verabreichung von 30 und 60 mg Ambroxol-HCl ergaben eine ausgezeichnet Langzeitwirkung hinsichtlich der Schnarchunterdrückung. Bei Verabreichung von Bromhexin beträgt der bevorzugte Wirkstoffbereich 8 bis 30 mg. Bei Gabe von Carbocistin wird bevorzugt eine Wirkstoffdosis von 200 bis 400 mg gewählt.

Zur Erzielung eine über die Nacht andauernden Langzeitwirkung ist es vorteilhaft, das erfindungsgemässe Antischnarchmittel als Retardform zu formulieren. Dazu wird der Wirkstoff zusammen mit Hilfsstoffen so formuliert, dass dieser nur sehr langsam freigegeben wird, was z.B. durch Einbettung desselben in eine sich sehr langsam auflösende Matrix erfolgen kann. Ausserdem ist es möglich, den Wirkstoff zusammen mit Hilfsstoffen zu Tabletten, Pellets, Granulat oder beliebigen sphäroiden Teilchen oder Komprimaten zu formen, die dann mit einem entsprechenden Überzug versehen werden, der eine langsame Freisetzung des Wirkstoffes im Magen und/oder Darmtrakt zur Folge hat. Aus galenischer Sicht soll der Wirkstoff für eine Retardform so formuliert werden, dass keine Änderung der Resorptionsgeschwindigkeit im resorptionsfähigen Teil des Magen-Darm-Traktes erfolgt. Hierbei hat es sich in manchen Fällen bewährt, den Wirkstoff mit einem Emulgator zu vermischen und gegebenenfalls mit einem säureunlöslichen Überzug zu versehen, so dass dann im Darmsaft der Wirkstoff in solubilisierter Form freigesetzt wird.

Ausserdem ist es möglich, eine Zwei-Phasen-Zubereitung zu verabreichen, wonach z.B. eine Mantel-Tablette, die 60 mg Ambroxol-HCl enthält, über einen isolierten Kern von 30 mg Ambroxol-HCl verfügt, der erst nach 3 bis 4 Stunden frei wird, während sich der Mantel sogleich auflöst.

Sofern die vorstehenden genannten Sekretolytika im Magen bzw. Darmtrakt nicht in ausreichendem Masse resorbiert werden, empfiehlt es sich, auch den Wirkstoff in einem Gemisch mit resorptionssteigernden bzw. die pH-Verhältnisse günstig beeinflussenden Substantzen anzuwenden.

Eine derartige galenische Formulierung wird z.B. bei der Anwendung von Bromhexin gewählt. Hierzu ist es vorteilhaft, den Wirkstoff im Gemisch mit einer Säure bzw. einer sauren Substanz in die Form von Granulat, Tablettenkernen, Mikrokapseln, etc. zu bringen. Hierzu wird 1 Mol Wirkstoff mit 1 bis 60 Mol, bevorzugt mit 5 bis 30 Mol, Säure oder saurer Substanz vermischt. Solche Formulierungen sind in der DE—A— 31 26 703 ausführlich beschrieben, auf welche hier ausdrücklich Bezug genommen wird. Bevorzugt ist es, die den Wirkstoff enthaltenden Zubereitungen in Hartgelatine-Kapseln abzufüllen, deren Zersetzung und damit die Resorption des Wirkstoffes im Darmtrakt erfolgt. Hierzu werden die sekretolytischen Wirkstoffe in eine Zubereitungsform gebracht, die von einem säureunlöslichen, darmsaftlöslichen Lack umgeben ist. Besonders geeignete säureunlösliche, darmsaftlösliche Substanzen sind in der vorstehend genannten DE—A—31 26 703 beschrieben, auf welche hier Bezug genommen wird. Insbesondere sind zu nennen: Methacrylsäure-Methacrylsäureester-Mischpolymerisat, Hydroxypropylmethyl-cellulosephthalat oder Celluloseacetatsuccinat.

Die Massnahme, die den Wirkstoff enthaltende Zubereitung mit einem säurenlöslichen, darmsaftlöslichen Lack bzw. einer entsprechenden Hartgelatine-Kapsel zu umgeben, ist besonders geeignet bei der Herstellung von Retardformen. Auf diese Weise wird der zum Teil sehr guten Löslichkeit der erfindungsgemäss verwendeten Sekretolytika Rechnung getragen, die dann nur langsam freigesetzt werden. Auf diese Weise kann erreicht werden, dass der sekretolytische Wirkstoff über Stunden in gelöster, resorptionsfähiger Form zur Verfügung steht.

Insbesondere eignen sich als Antischnarchmittel gemäss der Erfindung die im Handel erhältlichen Retardformen der Sekretolytika Bromhexin und Ambroxol, die unter den Markennamen Bisolvon® und Mucosolvan® bekannt sind.

Die Herstellung von nicht retardierenden Tabletten, Dragees, etc. erfolgt nach an sich bekannten Verfahren. Besonders vorteilhaft ist die Verabreichung von mit einer Lacksicht umgebenen Mikrokapseln, da die Aufteilung der Wirkstoffdosis auf viele hundert selbständige, kleine Retardformen erfolgt und dadurch eine gleichmässige Wirkstofffreigabe gewährleistet wird.

Den erfindungsgemässen Mitteln können darüber hinaus schleimhautverträgliche Substanzen, welche die Bildung von Mikrorissen verhindern oder die Beseitigung oder Auflösung von Störsubstanzen bestünstigen, zugegeben werden.

Ausserdem können dem erfindungsgemässen Antischnarchmittel auch etherische Öle sowie Gemische von etherischen Ölen zugegeben werden. Hiervon ist insbesondere bevorzugt der Einsatz von Ol. Thymi, Ol. Anisi, Ol. Eucalypti, Ol. Camomille, Ol. Menthae, Ol. Terebinthiniae.

Im weiteren kann das erfindungsgemässe Antischnarchmittel Substanzen enthalten, die die sekretolytische Wirkung des eingesetzen Wirkstoffes unterstützen bzw. sich anderweitig vorteilhaft auf die Beschaffenheit der Nasen-Rachenschleimhaut auswirken. Hierzu sind insbesondere die Substanzen Vitamin A und Vitamin E zu nennen. Die Anwesenheit von Vitamin A bewährt sich aufgrund seiner regenerativen Beeinflussung von Gewebsformationen. Vitamin E wirkt jeglicher Degeneration entgegen, entgiftet, steigert die Abwehr und regeneriert dan mesenchymalen Bereich, wodurch es vegetativ zu einer Tonussteigerung kommt. Im Falle der Zugabe von Vitamin A zu dem erfindungsgemässen Antischnarchmittel erfolgt dies in einer Menge von ca. 15.000 bis 30.000 IE/ml. Vitamin E kann z.B. als Acetat den erfindungsgemässen Mitteln in einer Menge von ca. 20 bis 200 mg/ml zugegeben werden.

Im folgenden werden spezielle Zubereitungsformen des erfindungsgemässen Antischnarchmittels für die orale Anwendung aufgeführt. Die nachfolgenden Rezepturen stellen einige bevorzugte Beispiele aus der Fülle möglicher Formulierungen dar.

Formulierung 1

Es wird folgende Zusammensetzung zur Herstellung von Kapseln bereitet:

| | |
|---|---|
| Ambroxol-hydrochlorid | 10,0 g |
| Maisstärke getr. | 24,0 g |
| Aerosil 200 | 0,4 g |

Die Inhaltstoffe werden gemischt, durch ein Sieb von 0,75 mm gegeben und in ca. 200 Hartgelatine-Kapseln mit einem Kapsel-Füllgewicht von 170 mg abgefüllt.

Formulierung 2

Es werden Tabletten der folgenden Zusammensetzung hergestellt:

1 Tablette enthält:

| | |
|---|---|
| Carbocistein | 300,0 mg |
| Milchzucker | 50,0 mg |
| Maisstärke | 50,0 mg |
| Polyvinylpyrrolidon | 2,0 mg |
| Magnesiumstearat | 1,0 mg |

Der Wirkstoff wird mit Milchzucker und Maisstärke gemischt, mit einer wässrigen PVP-Lösung befeuchtet, das Gemisch durch ein 1,5 mm-Sieb gegeben, und das erhaltene Granulat getrocknet. Nach Zumischen des Schmiermittels werden die Tabletten gepresst.

Formulierung 3

Zur Herstellung eines oralen Antischnarchmittels in Retardform werden folgende Pellets hergestellt:

Zunächst bereitet man unter Verwendung von alkoholischer Polyvinylpyrrolidonlösung, Weinsäure, Talkum und dem Wirkstoff Bromhexin Pellets von ca. 0,8 mm Duchmesser, die ca. 20% Bromhexin und ca. 75% Weinsäure enthalten.

Die vorstehend genannten, getrockneten Pellets werden dann in einem Dragierkessel mit einer Lösung von Methacrylsäure-Methacrylsäureester-Mischpolymerisat (Säurezahl 200 bis 300) und Hydroxypropylmethylcellulosephthalat in Isopropanol/Acetat in einem Verhältnis Pellet/Überzug 10:1 besprüht, wobei als Weichmacher Triacetin eingesetzt wird.

Die bei Anwendung der erfindungsgemässen Mittel durchgeführten Versuche am Menschen haben gezeigt, dass dieselben keinerlei Unverträglichkeiten hervorrufen, auch nicht bei Anwendung über eine längere Zeit, weil die Komponenten in den angegebenen Konzentrationen nicht toxisch sind und als solche in der Rhinologie bereits angewendet werden.

**Patentansprüche**

1. Verwendung einer sekretolytisch und/oder sekretoproduktiv werden Substanz neben üblichen pharmazeutisch verträglichen Trägersubstanzen oder Streck- bzw. Verdünnungsmitteln zur Herstellung eines Mittels zur therapeutischen Behandlung gesundheitsschädigenden Schnarchens in einer oralen Verabreichungsform, die einen intensiven Kontakt des Wirkstoffes mit der Rachenschleimhaut ausschliesst.

2. Verwendung nach Anspruch 1, wobei das Antischnarchmittel einen wirksamen Gehalt an Bromhexin, Ambroxol, Eprazinon, einem Saponin, N-Acetyl-L-cystein und/oder Carbocistin enthält.

3. Verwendung nach Anspruch 1 oder 2, wobei das Antischnarchmittel in Form von Tabletten, Dragees, Kapseln, Mikrokapseln oder als Granulat vorliegt.

4. Verwendung nach den Ansprüchen 1 bis 3, wobei die Gesamtwirkstoffdosis zwischen 5 und 500 mg liegt.

5. Verwendung nach Anspruch 1, wobei das Antischnarchmittel als Zusatz neben dem Wirkstoff eine Säure bzw. saure Substanz enthält.

6. Verwendung nach Anspruch 5, wobei auf 1 Mol Wirkstoff 1 bis 60 Mol Säure bzw. saure Substanz entfallen.

7. Verwendung nach Ansprüchen 1 bis 5, wobei eine den Wirkstoff enthaltende feste Zubereitungsform von einem säureunlöslichen, darmsaftlöslichen Lack umgeben wird.

8. Verwendung nach Ansprüchen 1 bis 5, wobei eine den Wirkstoff enthaltende feste Zubereitung in Hartgelatinekapseln abgefüllt wird.

9. Verwendung nach einem oder mehreren der Ansprüche 1 bis 8, wobei das Antischnarchmittel ein etherisches Öl bzw. ein Gemisch von etherischen Ölen enthält.

10. Verwendung nach Anspruch 9, wobei das Antischnarchmittel ein etherisches Öl aus der Gruppe Ol. Thymi, Ol. Ainsi, Ol. Eucalypti, Ol. Camomille, Ol. Terebinthiniae, Ol. Menthae enthält.

11. Verwendung nach Anspruch 1 bis 10, wobei das Antischnarchmittel neben dem Wirkstoff eine oder mehrere der Substanzen aus der Gruppe Vitamin A und Vitamin E enthält.

12. Verwendung nach einem oder mehreren der Ansprüche 1 bis 11, wobei das oral anzuwendende Antischnarchmittel als Retardform formuliert ist.

13. Verwendung nach Anspruch 12, wobei das Antischnarchmittel 20% Bromhexin-hydrochlorid, 75% Weinsäure und 5% Talkum umfasst.

14. Verwendung nach einem oder mehreren der Ansprüche 1 bis 13, wobei das oral anzuwendende Antischnarchmittel folgende Zusammensetzung hat:

Ambroxol-hydrochlorid 50 bis 150 mg

Maisstärke 120 g

Aerosil 200 2 g

15. Verwendung einer sekretolytisch und/oder sekretoproduktiv wirkenden Substanz gegen störendes Schnarchen, in einer oralen Verabreichungsform, die einen intensiven Kontakt des Wirkstoffes mit der Rachenschleimhaut ausschliesst.

**Revendications**

1. Utilisation d'une substance sécrétolytique et/ou à action sécrétoproductive, en plus des substances supports pharmaceutiquement compatibles usuelles, ou des agents amolissants ou diluants usuels, pour la fabrication d'un agent servant au traitement thérapeutique du ronflement qui est nuisible à la santé, sous une forme destinée à une administration par voie orale, qui exclut tout contact intensif de la substance active avec la muqueuse du pharynx.

2. Utilisation selon la revendication 1, dans laquelle l'agent contre le ronflement contient une teneur active en bromhéxine, ambroxol, éprazine, une saponine, de la N-acétyl-L-cystine et/ou de la carbocistine.

3. Utilisation selon les revendications 1 à 3, dans laquelle l'agent contre le ronflement se présente sous forme de pilules, dragées, capsules, microcapsules ou de granulé.

4. Utilisation selon la revendication 1, dans laquelle le dosage total en substances actives est situé entre 5 et 500 mg.

5. Utilisation selon la revendication 1, dans laquelle l'agent contre le ronflement contient, en tant qu'additif à la substance active, un acide, respectivement une substance acide.

6. Utilisation selon la revendication 5, dans laquelle, pour une mole de substance active, il y a de 1 à 60 moles d'acide ou de substance acide.

7. Utilisation selon les revendications 1 à 5, dans laquelle l'une des formes de préparation solide contenant la substance active est entourée d'un vernis insoluble dans de l'acide, soluble dans le suc intestinal.

8. Utilisation selon les revendications 1 à 5, dans laquelle l'agent l'une des formes de préparation solide contenant la substance active est insérée dans des capsules en gélatine dure.

9. Utilisation selon une ou plusieurs des revendications 1 à 8, dans laquelle l'agent contre le ronflement contient une huile éthérique, ou un mélange d'huiles éthériques.

10. Utilisation selon la revendication 9, dans laquelle l'agent contre le ronflement contient une huile éthérique du groupe constitué par l'huile de thym, d'anis, d'eucalyptus, de camomille, de thérébenthine, de menthe.

11. Utilisation selon la revendications 1 à 10, dans laquelle l'agent contre le ronflement contient, en plus de la substance active, une ou plusieurs substances du groupe de la vitamine A et de la vitamine E.

12. Utilisation selon une ou plusieurs des revendications 1 à 11, dans laquelle l'agent contre le ronflement à administrer par voie orale est formulé sous forme à effet retardé.

13. Utilisation selon la revendication 12, dans laqelle l'agent contre le ronflement contient 20% d'hydrochlorure de bromhéxine, 75% d'acide tartrique et 5% de talc.

14. Utilisation selon une ou plusieurs des revendications 1 à 13, dans laquelle l'agent contre le ronflement à administrer par voie orale présente la composition suivant:

hydrochlorure d'ambroxol
50 à 150 mg

amidon de maïs 120 g

aérosil 200 2 g

15. Utilisation d'une substance sécrétolytique et/ou à action, sécrétoproductive contre un ronflement perturbateur, sous une forme destinée à une administration par voie orale, qui exclut tout contact de la substance active avec la muqueuse du pharynx.

**Claims**

1. Use of a secretolytically and/or secretoproductively active substance together with the usual pharmaceutically compatible carrier substances or extenders and/or thinning agents for the preparation of an agent for the therapeutic treatment of snoring which is injurious to health in the form for oral administration which prevents intensive contact of the active ingredient with the pharyngeal mucous membrane.

2. Use according to claim 1, wherein the antisnoring agent has an efficaceous content of bromhexine, ambroxol, eprazinone, a saponin, N-acetyl-L-cysteine and/or carbocistine.

3. Use according to claim 1 or 2, wherein the antisnoring agent is in the form of tablets, dragees, capsules, microcapsules or as a granulate.

4. Use according to claims 1 to 3, wherein the total dose of active ingredient is between 5 and 500 mg.

5. Use according to claim 1, wherein the antisnoring agent contains an acid or acidic substance as an additive apart from the active ingredient.

6. Use according to claim 5, wherein 1 to 60 moles acid or acidic substance are apportioned to 1 mole active ingredient.

7. Use according to claims 1 to 5, wherein a solid form of preparation containing the active ingredient is surrounded by an acid-insoluble, intestinal juice-soluble coating.

8. Use according to claims 1 to 5, wherein hard gelatin capsules are filled with a solid preparation containing the active ingredient.

9. Use according to one or more of claims 1 to 8, wherein the antisnoring agent contains an essential oil or a mixture of essential oils.

10. Use according to claim 9, wherein the antisnoring agent contains an essential oil selected from the group comprising Ol. Thymi, Ol. Anisi, Ol. Eucalypti, Ol. Camomille, Ol. Terebinthiniae, Ol. Menthae.

11. Use according to claims 1 to 10, wherein the antisnoring agent contains in addition to the active ingredient one or more of the substances selected from the group comprising vitamin A and vitamin E.

12. Use according to one or more of claims 1 to 11, wherein the antisnoring agent to be used orally is formulated as a retard form.

13. Use according to claim 12, wherein the antisnoring agent includes 20% bromhexine hydrochloride, 75% tartaric acid and 5% talc.

14. Use according to one or more of claims 1 to 13, wherein the antisnoring agent to be used orally has the following composition:

| | |
|---|---|
| ambroxol hydrochloride | 50 to 150 mg |
| maize starch | 120 g |
| Aerosil 200 | 2 g. |

15. Use of a secretolytically and/or secretoproductively active subtstance against disturbing snoring, in a form for oral administration which prevents intensive contact of the active ingredient with the pharyngeal mucous membrane.